# EUROPEAN PATENT APPLICATION

(11) **EP 1 627 615 A2**
(43) Date of publication of application: **22.02.2006**
(21) Application number: 05107554.7
(22) Date of filing: 17.08.2005
(51) Int. Cl.: A61F 2/42, A61L 27/52

(54) **Modular joint replacement implant with hydrogel surface**

(30) Priority: 18.08.2004 US 602316 P
(71) Applicant: Arthrex, Inc., Naples, Florida 34108-1945 (US)
(72) Inventor: Schmieding, Reinhold, Naples, Florida 34108 (US)
(74) Representative: Vittorangeli, Lucia

(57) **Abstract**

An endoprosthetic joint implant (4,6) includes a fixation element (10,20) and a hydrogel hinge portion (8,18). The fixation element (10,20) supports the hydrogel hinge portion (8,18) on a resected bone-end, for example, for use in surgical joint replacement. The fixation element (10,20) features a stem (10,20) made of titanium. The stem (10,20) is threaded for insertion distally into an intramedullary canal. The hydrogel hinge portion (8,18) connects to the stem (10,20) using a snap-fit arrangement. The hydrogel is available in various surface configurations and can articulate against a natural joint surface or a synthetic joint surface. A total joint-replacement implant (2) is provided by attaching a second stem to an opposite side of the hinge for insertion into bone on the opposed side of the articulation.

## Description

### BACKGROUND OF THE INVENTION

### 1. Field of the Invention:

The present invention relates to endoprosthetic articular joint implants, and more specifically to methods and apparatus for performing surgical joint repair and replacement procedures using a modular joint implant with hydrogel articular surfaces fixed by an anchoring stem.

### 2. Description of the Related Art:

Prosthetic devices for joint repair and replacement in humans and other skeletal beings are known. Joint function can be lost due to fracture or damaged joint surfaces, for example. Joints compromised in these ways typically have suffered injury or disease. Repair often entails endoprosthetic implants installed in place of resected bone-ends to replace the damaged joint implant and restore joint function.

Joint-replacements are known that generally include an intramedullary stem anchoring a curved joint surface. U.S. Pat. No. 5061288 to Berggren et al. Berggren et al. discloses a joint implant that features a surface-replacing portion attached to the end of an axial support. Bristles extend radially from the axial support sufficient to be urged proximally upon insertion to the canal. The bristles consequently are biased against removal of the implant, thereby acting as barbs to anchor the surface-replacing portion of the implant on the end of resected bone. A joint-surface material features a dove-tail groove that engages a base with a corresponding pin attached to the axial support.

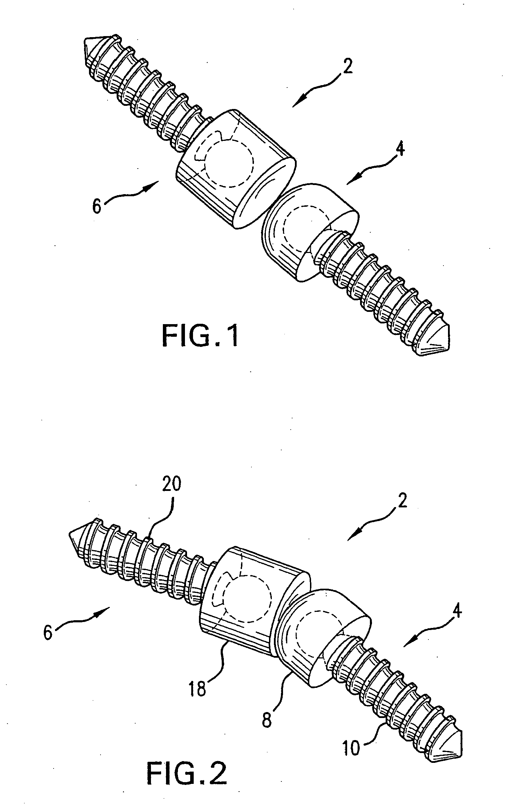

French patent document 2,605,878 in the name of Condamine discloses a two part joint prosthesis. Referring to FIGS. 1-3 of Condamine, the implant features an articulating surface portion that is adhered to a ribbed intramedullary stem. Another French patent document, no. 1122634 in the name of van Steenbrugghe discloses implants with coated spherical ends. The coating can include a polymer, such as Plexiglas, Lucite, polyamides (nylon), polyesters, and fluorethylenes, for example.

U.S. Patent No. 5,314,486 to Zang et al. discloses a joint implant having an insertable bearing-surface. A non-bearing intermediate portion adds complexity and an additional implant that increases the number of potential problems that could lead to failure.

There is a need in the prior art for a simple but effective modular joint prosthesis that includes an anchoring stem and interchangeable, snap-to-fit articulating surfaces with improved properties and materials.

### SUMMARY OF THE INVENTION

The invention provides a modular endoprosthetic implant that features a hydrogel portion that simulates an articular surface. The hydrogel portion is supported by a fixation element that secures the modular endoprosthetic implant in place.

In an exemplary embodiment, the hydrogel portion locks onto a fixation element that includes a threaded anchoring-stem. The threaded anchoring-stem allows insertion into an intramedullary canal, for example, to achieve implant fixation. Other forms of fixation including without limitation, press-fit, interference fit, adhesives, etc. are contemplated.

The hydrogel portion is secured to the exemplary fixation element with the threaded stem by a snap-fit arrangement. The fixation element features a ball that extends from the fixation element on a side opposite the threaded stem. The hydrogel portion is part of an articular element that features a socket corresponding to the ball. The articular element is secured to the fixation element by way of a snap-on fit of the ball into the socket. The articular element articulates against normal opposing articulating cartilage surfaces, or against an opposing implant that provides a complementary replacement articular surface.

Other features and advantages of the present invention will become apparent from the following description of exemplary embodiments of the invention with reference to the accompanying drawings.

### BRIEF DESCRIPTION OF THE DRAWINGS

FIG. 1 is a perspective illustration of a modular implant with molded articular modules represented to reveal within a ball-end of a fixation element including a screw stem according to the present invention;

FIG. 2 is a perspective illustration of the modular implant of FIG. 1, with the ball and socket assemblies engaged to represent a working joint;

FIG. 3 is a mechanical drawing of a modular joint implant according to the present invention with the molded articular modules shown in cross section;

FIG. 4 is a mechanical drawing showing an exploded view of a modular joint implant according to the present invention;

FIG. 5 is a perspective drawing of a modular joint implant according to the present invention showing the prosthetic joint in an articulated position;

FIG. 6 is a perspective drawing of a modular joint implant according to the present invention;

FIG. 7 is a mechanical drawing showing an exploded view of a modular joint implant according to the present invention with the molded articular modules shown in cross section;

FIG. 8 illustrates a modular joint implant according to the present invention in which an anchoring stem is secured to a male connector insert-molded within the articular module;

FIG. 9 illustrates a modular joint implant according to the present invention in which an anchoring stem is secured to a female connector insert-molded within the articular module;

FIG. 10 illustrates a modular joint implant according to the present invention in which opposing anchor posts are secured to connectors insert molded within a central articular module; and

FIG. 11 illustrates the modular joint implant of FIG. 10 installed to replace a joint in a finger.

### DETAILED DESCRIPTION OF THE INVENTION

Referring to FIGS. 1-7, an endoprosthetic total joint replacement system 2 according to the present invention is shown. Total joint replacement system 2 includes a cooperating pair of modular joint implants 4, 6.

Implant 4 includes a ball-shaped articular head portion 8 and a threaded stem 10. Head portion 8 is formed from molded hydrogel. Threaded stem 10 is titanium. A spherical ball-end 12 of the threaded stem 10 snap-fits into a socket 14 formed in ball-shaped head portion 8.

Similarly, implant 6 includes a cup-shaped molded hydrogel articular head portion 18 and a threaded stem 20. A spherical ball-end 22 of threaded stem 20 snap-fits into a socket 24 formed in cup-shaped head portion 18.

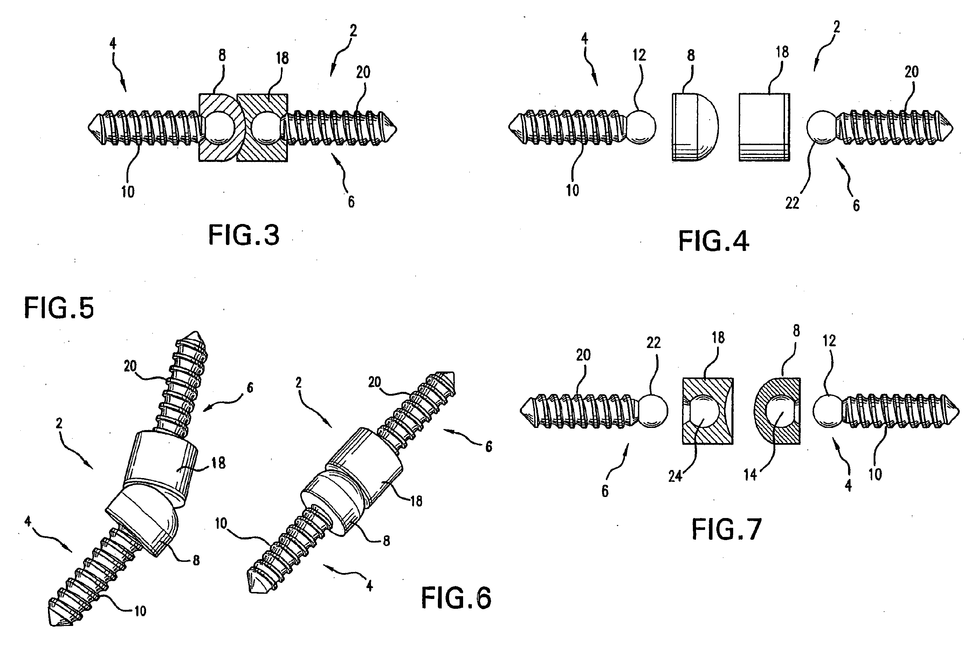

As can be seen more clearly in FIGS. 3 and 7, sockets 14, 24 formed in respective head portions 8, 18 have a cross-section substantially identical to the cross-section of corresponding spherical ball-ends 12, 22 of threaded stems 10, 20. Accordingly, an outer circumference of the ball-ends 12, 22 is substantially identical to a circumference of the spherical sockets 14, 24. The openings in sockets 14, 24 have a minimum circumference that is substantially smaller than the circumference of the ball-ends. Advantageously, the socket openings are provided with a flared configuration, shown most clearly in FIG. 7. The flared configuration enables guiding the head portions 8, 18 over the respective ball-ends 12, 22. The hydrogel material of the head portions 8, 18 is sufficiently elastic and features enough resilience that as the head portions 8, 18 are urged onto the corresponding ball-ends 12, 22, the hydrogel material stretches to allow entry of the ball-ends 12, 22 into the sockets 14, 24. Once the ball-ends 12, 22 are fully situated within the sockets 14, 24, the resilient hydrogel material will re-assume an original shape to provide a "snap-fit" of the ball-ends 12, 22 within head portions 8, 18.

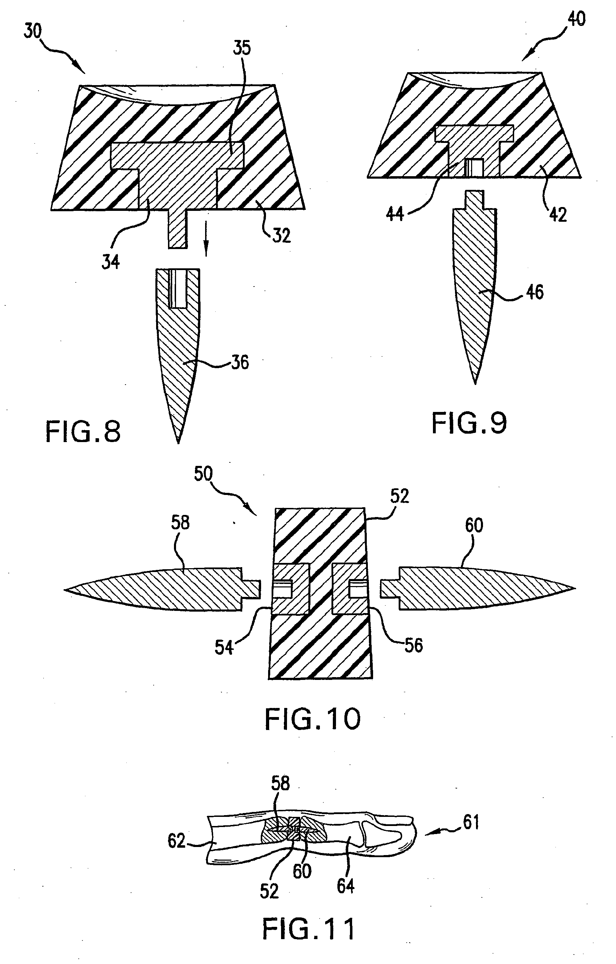

Referring to FIG. 8, a modular joint assembly 30 according to an alternative embodiment of the present invention includes an articular element 32 molded of hydrogel and into which a male connector 34 has been installed, preferably by insert-molding the hydrogel around connector 34. The head portion 32 features hydrogel as the majority of the implant. The head portion 32 can be at least 50% hydrogel by volume. Connector 34 can include retention features such as extension 35 that enhance retention of the connector 34 within the molded hydrogel. An anchoring stem 36 includes a corresponding female socket that receives the male pin of connector 34 being installed as indicated by the arrow in FIG. 8. Although a cup-shaped head portion 32 having a tapered external appearance is illustrated in FIG. 8, other shapes of head portions can be similarly-attached to stem 36. Further, although a tapered pin is shown in FIG. 8, stem 36 can be threaded, for example, or can be a flat body adhered to a bone-end, and is not limited by the illustration.

Referring to FIG. 9, a modular joint assembly 40 according to an alternative embodiment of the present invention includes a hydrogel-molded articular element 42 into which a female connector 44 has been insert-molded. A pin of an anchor post 46 is received into the female connector 44. Further advantages of assembly 40 and its features will be understood from corresponding descriptions provided above in connection with FIG. 8.

Referring to FIGS. 10 and 11, a modular total-joint replacement system 50 is illustrated. Joint assembly 50 includes a molded-hydrogel articular-hinge 52 into which two connectors 54 and 56 have been insert-molded. Connectors 54, 56 receive anchoring stems 58, 60 for fixation within a finger 61. Although FIG. 10 shows female connectors on the articular hinge 52 and corresponding male connectors on anchoring stems 58, 60, the reverse and other arrangements also could be used.

Referring more specifically to FIG. 11, anchoring stems 58, 60 are installed into intramedullary canals of finger bones 62, 64. The articular hinge module 52, formed of a hydrogel, for example, provides flexibility sufficient to function as a proximal interphalangeal joint, for instance, between proximal phalange 62 and middle phalange 64 of finger 61.

The molded hydrogel articular portions 8, 18, 32, 42, 52 of the present invention are formed in an exemplary embodiment of Salubria, a trademarked product of SaluMedica LLC. Hydrogels are colloidal gels in which water is the dispersion medium. The Salubria hydrogel material in particular is described in U.S. Patent Nos. 5,981,826 and 6,231,605. The hydrogel contains water in similar proportions to human tissue and is similar in its mechanical and physical properties. The hydrogel is an organic polymer-based biomaterial known to be highly biocompatible. Used in articular applications, the hydrogel material is soft and compliant like human tissue, and is exceptionally wear resistant and strong, making it an exemplary implant suitable for many medical applications.

The anchor posts 10, 20, 36, 46, 58, 60 are composed of titanium according to an exemplary embodiment, though other strong, biocompatible implant materials are known, including other metals, metal composites and alloys, and ceramics, for example.

Although the present invention has been described in connection with preferred embodiments, many modifications and variations will become apparent to those skilled in the art.

## Claims

1. An endoprosthetic joint implant comprising:
a fixation element having a distal portion and a proximal portion;
an attachment part formed on the distal portion; and
an articular element disposed on the proximal portion, the articular element comprising hydrogel contoured to engage a complementary articular surface.

2. An endoprosthetic joint implant as in claim 1, wherein the attachment part is a threaded screw.

3. An endoprosthetic joint implant as in claim 1, wherein the articular element consists of hydrogel.

4. An endoprosthetic joint implant as in claim 1, wherein the articular element is more than 50% hydrogel by volume.

5. An endoprosthetic joint implant as in claim 1, wherein exposed outer surfaces of the articular element comprise hydrogel.

6. An endoprosthetic joint implant as in claim 1, wherein the attachment part is an intramedullary stem.

7. An endoprosthetic joint implant as in claim 1, wherein the attachment part and the fixation element are unitary.

8. An endoprosthetic joint implant as in claim 1, wherein the articular element provides a socket into which the fixation element is received.

9. An endoprosthetic joint implant as in claim 1, wherein the fixation element comprises a ball and the articular element comprises a socket, the ball having a circumference larger than an opening to the socket.

10. An endoprosthetic joint implant as in claim 9, wherein the ball is supported on a neck having a circumference less than or equal to the circumference of the ball.
